# EUROPEAN PATENT SPECIFICATION

(11) **EP 4 374 893 B1**
(45) Date of publication and mention of the grant of the patent: **21.10.2026**
(21) Application number: 24167844.0
(22) Date of filing: 25.09.2020
(51) Int. Cl.: A61M 5/20, A61M 5/14, A61M 5/142

(54) **DRIVING PISTON SET OF AN INJECTOR AND CONVERSION MECHANISM HAVING THE SAME**
ANTRIEBSKOLBENSATZ EINES INJEKTORS UND UMRÜSTUNGSMECHANISMUS DAMIT
ENSEMBLE DE PISTON D'ENTRAÎNEMENT D'UN INJECTEUR ET MÉCANISME DE CONVERSION LE COMPRENANT

(43) Date of publication of application: 29.05.2024
(62) Document number(s) of the earlier application(s) in accordance with Art. 76 EPC: 20954567.2 / 4 218 858
(73) Proprietor: CC Biotechnology Corporation, Tainan City, Taiwan 70955 (TW)
(72) Inventor: YEH, CHIN-MIN, Tainan City (TW); WU, PIN-HAN, Tainan City (TW)
(74) Representative: Cabinet Chaillot

(56) References cited:
- WO-A1-2015/125563
- US-A1- 2008 097 375
- US-A1- 2012 118 138
- US-B2- 10 583 244

## Description

This application is a divisional application of European patent application No. 20954567.2, which is a European National Phase of PCT/CN2020/117746, filed on September 25, 2020.

### 1. Field of the Invention

The present invention relates to an injector used for subcutaneous injection of a pharmaceutical agent into a human body, especially to a driving piston set of the injector and a conversion mechanism having the driving piston set.

### 2. Description of the Prior Art(s)

A conventional injector for subcutaneous injection of a pharmaceutical agent into a human body has a manually or electrically operated injection module connected to a medicament container, so a needle assembly of the injection module can pierce the human body for subcutaneous injections. Besides, in order to prevent discomfort of the human body caused by piercing and removal of needle many times when multiple subcutaneous injections are to be performed on the human body, a needle assembly of the conventional injector has a soft needle and a hard needle. Since the soft needle is soft and thus is difficult to directly pierce the human body, the soft needle pierces the human body with the hard needle mounted inside the soft needle. Afterward, the hard needle is drawn out from the soft needle and the soft needle is connected to the injection module and the medicament container for injections. The soft needle is able to stay longer in the human body for multiple injections.

Based on safety regulations for the pharmaceutical agent injection, some conventional injectors are available for being reused for a limited number of times. However, when the limited number of times of reuses is reached, an entire set of the conventional injector has to be discarded and cannot be reused. Moreover, a combination structure of the conventional injector is fixed and a driving mechanism thereof is directly connected to an injection module and would come into contact with the pharmaceutical agent to be injected. For safety and hygiene reasons, it is difficult to reuse part of the mechanism of the conventional injector and further improvements are necessary.

A conventional DRUG SOLUTION INJECTION DEVICE, as disclosed in WO 2015/125563 A1, includes a main body with a drug solution container. The drug solution container is provided with: a cylinder with an introduction portion introducing drug solution into the cylinder; a piston provided inside the cylinder; and an operating member connected to the piston and being for operating the piston from the outside of the cylinder. A dynamic force-supplying mechanism comprises a pump capable of supplying dynamic force for driving the drug solution out of the drug solution container by suction of drug solution stored inside a storing chamber formed between the cylinder and the piston. The operating member can be detached from the piston. The size of the overall drug solution injection device can be decreased while reducing the chances of a medical practitioner coming into contact with the drug solution.

A conventional SYSTEM, APPARATUS AND METHODS FOR FLUID INFUSION INTO A BODY, as disclosed in US 10,583,244 B2, discloses a portable fluid infusion device comprising a disposable part (DP) and a reusable part (RP). The DP comprises a base, a first reservoir for storing infusion fluid, a second reservoir for administering the infusion fluid to the patient in accurate fashion, and an exit port. The second reservoir is less than or equal to the first reservoir in length. The RP comprises a first compartment configured to receive the first reservoir, a second compartment configured to receive the second reservoir, and a gasket for sealing a junction between the second reservoir and the second compartment upon connection of the RP and the DP. A first check valve is disposed between the first reservoir and the second reservoir, such that fluid flow is only from the first reservoir to the second reservoir. A second check valve is disposed between the second reservoir and the exit port such that fluid flow is only from the second reservoir to the exit port.

The main objective of the present invention which is defined in the independent claim, is to provide a driving piston set and a conversion mechanism of an injector to solve the problem that after being used, an entire set of a conventional injector with fixed structure has to be discarded due to safety and hygiene reasons, which results in waste, and a driving mechanism of the conventional injector would come into contact with the pharmaceutical agent to be injected and thus is difficult to be reused.

The driving piston set of the injector is configured to be used in the injector and be connected to a driving mechanism. The driving piston set has a piston cylinder, a valve, and a piston rod. The piston cylinder has a piston room and a valve connecting end segment defined on an end of the piston cylinder. The valve is assembled to the valve connecting end segment of the piston cylinder, and has an input one-way valve being only opened in a direction towards the piston cylinder and an output one-way valve being only opened in a direction outward from the piston cylinder. The piston rod is mounted in the piston cylinder and being linearly movable, and is detachably connected to the driving mechanism. An inner cylinder wall of the piston cylinder has at least one sliding groove. An outer annular surface of the piston rod has at least one sliding block. Each of the at least one sliding block is mounted in a corresponding one of the at least one sliding groove for guiding the piston rod to move linearly in the piston cylinder.

**In** the driving piston set of the injector as described, the driving piston set is mounted in a conversion mechanism of the injector and is connected to a medicament container containing medicament and a needle output mechanism having a needle set via the conversion mechanism, such that the driving mechanism drives the piston rod in the piston cylinder to draw the medicament from the medicament container and to inject the medicament via the needle set of the needle output mechanism.

In the driving piston set of the injector as described, the piston rod is a hollow rod and has at least one L-shaped buckling groove, so the piston rod and the driving mechanism is assembled by rotating to engage and the piston rod is driven by the driving mechanism.

The conversion mechanism of the injector is configured to be used in the injector and be connected to a driving mechanism having a driving unit. The conversion mechanism has a conversion connecting base and a driving piston set. The conversion connecting base has an input adapter and an output adapter. The driving piston set is mounted in the conversion connecting base and has a piston cylinder, a valve, and a piston rod. The piston cylinder has a piston room and a valve connecting end segment defined on an end of the piston cylinder. The valve is assembled to the valve connecting end segment of the piston cylinder and has an input one-way valve and an output one-way valve. The input one-way valve is connected to the input adapter and is only opened in a direction from the input adapter to the piston cylinder. The output one-way valve is connected to the output adapter and is only opened in a direction from the piston cylinder to the output adapter. The piston rod is mounted in the piston cylinder and is linearly movable, and is detachably connected to the driving mechanism.

**In** the conversion mechanism of the injector as described: the conversion mechanism is assembled with a needle output mechanism having a needle set to form an injection module; the input adapter of the conversion mechanism is connected to a medicament container containing medicament; the output adapter of the conversion mechanism is connected to the needle output mechanism; and the driving mechanism drives the piston rod in the piston cylinder to draw the medicament from the medicament container to the needle output mechanism and to inject the medicament via the needle set of the needle output mechanism.

**In** the conversion mechanism of the injector as described: an inner cylinder wall of the piston cylinder has at least one sliding groove; and an outer annular surface of the piston rod has at least one sliding block, each of the at least one sliding block is mounted in a corresponding one of the at least one sliding groove for guiding the piston rod to move linearly in the piston cylinder.

In the conversion mechanism of the injector as described: the piston rod is a hollow rod and has at least one L-shaped buckling groove, so the piston rod and the driving mechanism is assembled by rotating to engage and the piston rod is driven by the driving mechanism.

In the conversion mechanism of the injector as described: the conversion connecting base has a base body and a valve base segment mounted on the base body and having a sleeving opening; the input adapter is mounted on a side of the valve base segment opposite to the sleeving opening; the output adapter is mounted in the base body; and the input adapter and the output adapter are connected to the sleeving opening.

In the conversion mechanism of the injector as described, the base body has a connecting board, and the connecting board has two clamping segments located respectively on two opposite sides of the connecting board and a hook segment, so the connecting board engages with the needle output mechanism.

In the conversion mechanism of the injector as described, the driving mechanism has a case, the driving unit is mounted in the case, a side of the case has a connecting sleeve segment detachably sleeved on the piston cylinder, and the driving unit is linearly movable in the connecting sleeve segment.

The driving piston set and the conversion mechanism as described have the following advantage. With the driving piston set being detachably connected to the driving mechanism and being driven by the driving mechanism, the medicament in the medicament container is conveyed to the needle set for injection. Moreover, with the conversion connecting base of the conversion mechanism being detachably connected to the medicament container to allow the driving piston set to be driven by the driving mechanism, the medicament in the medicament container is conveyed to the needle set for injection. Thus, the medicament container and the injection module relating to safety and hygiene in the injector are able to be used once and be disassembled and discarded after user, and would not reused. In addition, with combination structure of the conversion mechanism and the driving mechanism externally connected to the conversion mechanism, the driving mechanism does not come into contact with the medicament during injection. Therefore, the driving mechanism is able to be reused, so as to reduce unnecessary waste of the injector.

### IN THE DRAWINGS:

Fig. 1 is a perspective view of a preferred embodiment of an injector including a conversion mechanism having a driving piston set in accordance with the present invention;
Fig. 2 is a partially exploded perspective view of the injector in Fig. 1;
Fig. 3 is a perspective view of a medicament container of the injector in Figs. 1 and 2;
Figs. 4A and 4B are operational perspective views of an injection module of the injector in Figs. 1 and 2;
Fig. 5 is an exploded perspective view of the conversion mechanism of the injection module of the injector in Figs. 1, 2, and 4;
Fig. 6 is an exploded perspective view from another viewing angle of the conversion mechanism of the injection module of the injector in Figs. 1, 2, and 4;
Fig. 7 is a partial side view of the injector in Figs. 1 and 2;
Fig. 8 is a sectional side view cutting along line 1-1 in Fig. 7;
Fig. 9 is a sectional side view cutting along line 2-2 in Fig. 7;
Fig. 10 is an exploded perspective view of a needle output mechanism of the injection module in Figs. 1, 2, and 4;
Fig. 11 is an exploded perspective view from another viewing angle of the needle output mechanism of the injection module in Figs. 1, 2, and 4;
Fig. 12 is a plan view of the needle output mechanism of the injection module in Figs. 1, 2, and 4;
Fig. 13 is a sectional side view cutting along line 3-3 in Fig. 12;
Fig. 14 is a sectional side view cutting along line 4-4 in Fig. 12;
Fig. 15 is an exploded perspective view of a driving mechanism of the injector in Figs. 1 and 2;
Fig. 16 is an exploded perspective view from another viewing angle of the driving mechanism of the injector in Figs. 1 and 2;
Figs. 17A and 17B are operational views of the injector in Figs. 1 and 2, showing the medicament container being assembled to the injection module;
Figs. 18A and 18B are operational views of the injector in Figs. 1 and 2, showing a combination of the medicament container and the injection module being assembled to the driving mechanism;
Fig. 19 is a sectional side view of the injector in Figs. 1 and 2, showing the needle output mechanism of the injection module without a safety cover and a safety bolt;
Fig. 20 is an operational view of the needle output mechanism in Fig. 19, showing a needle output operation assembly being pressed and driving a needle set located in a connecting member to protrude out a base with a hard needle mounted in a soft needle;
Fig. 21 is an operational view of the needle output mechanism in Fig. 20, showing the needle output operation assembly being fixed in the base, and a needle base being detached from the needle output operation assembly to drive the hard needle to detach from the soft needle;
Fig. 22 is an operational view of the needle output mechanism in Fig. 20, showing a needle retraction operation member driving the soft needle to retract after injection;
Fig. 23 is an operational view (1) of the needle output mechanism in Fig. 21, showing the needle output mechanism cutting a diameter reducing segment of a connecting head segment of the connecting member during retracting the needle set; and
Fig. 24 is an operational view (2) of the needle output mechanism in Fig. 21, showing the needle output mechanism cutting the diameter reducing segment of the connecting head segment of the connecting member during retracting the needle set.

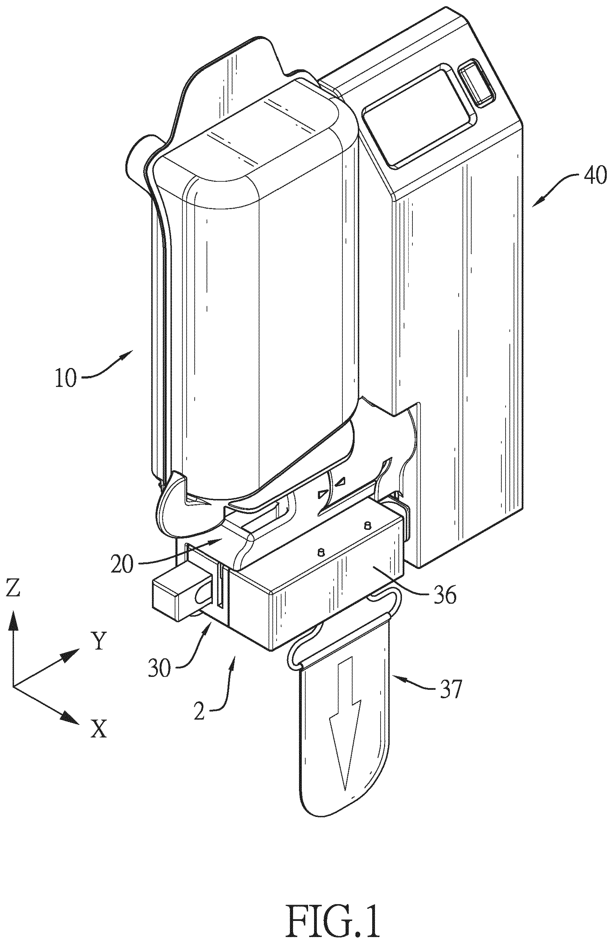

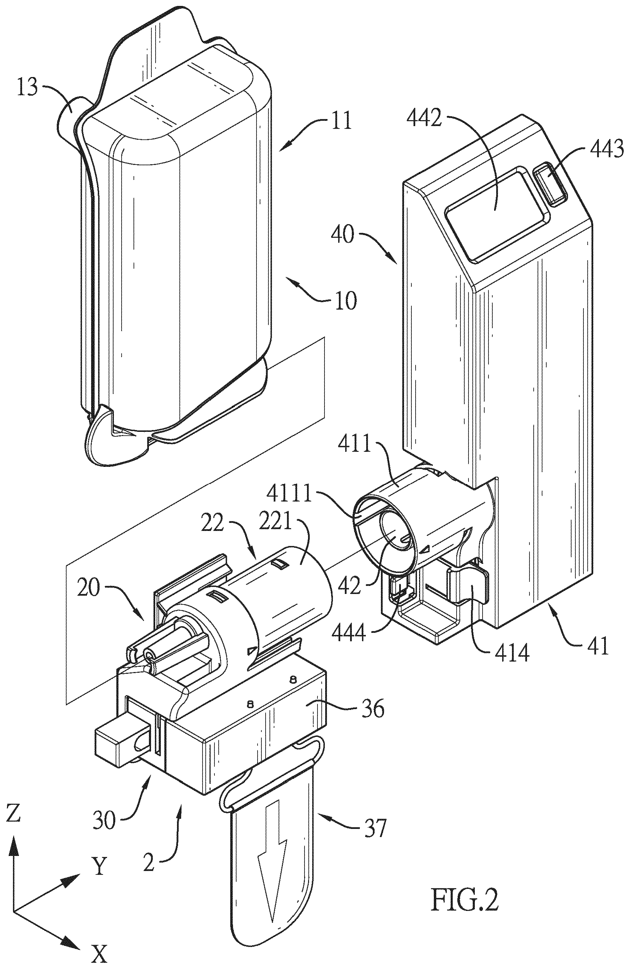

The present invention includes a driving piston set of an injector and a conversion mechanism of an injector. The conversion mechanism includes the driving piston set. With reference to Figs. 1 and 2, a preferred embodiment of the conversion mechanism including the driving piston set used in the injector is disclosed. The injector is located in a three-dimensional space having an X axis, a Y axis, and a Z axis. The injector has a front surface and a rear surface opposite to each other and is arranged along the X axis. The injector has a medicament container 10, an injection module 2, and a driving mechanism 40. In the preferred embodiment as shown in Fig. 2, the driving piston set 22 and the conversion mechanism 20 are part of the injection module 2. Specific structure of the driving piston set 22 and the conversion mechanism 20 as well as relevant components, which are connected to the driving piston set 22 and the conversion mechanism 20 of the present invention, in the injector are described as follows.

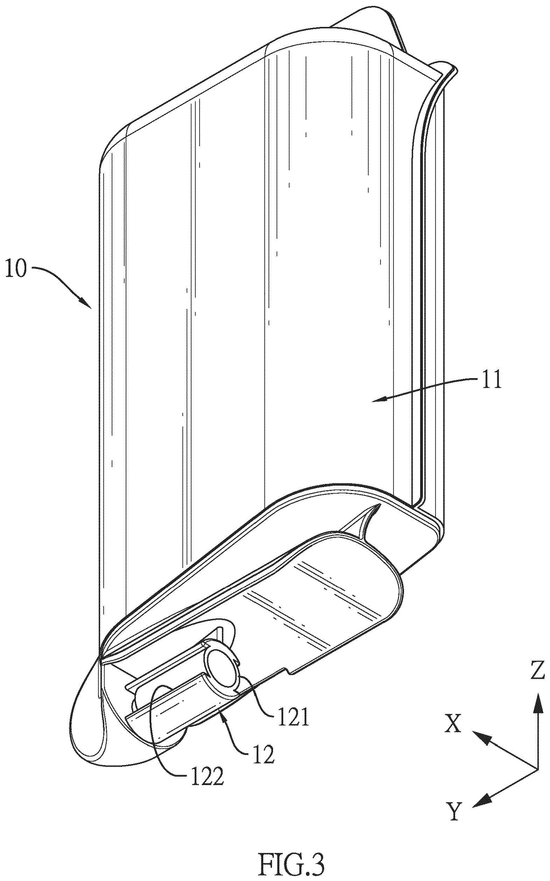

With reference to Figs. 1 to 3, the medicament container 10 is adapted to accommodate and seal a certain dose of medicament. The medicament container 10 has a container main body 11 and a container connecting port 12. The container main body 11 forms a space adapted to accommodate and seal the medicament. The container connecting port 12 is located on a side of the container main body 11 along a direction on the Z axis and is connected to an inner side of the container main body 11. The medicament filled in the container main body 11 can be output via the container connecting port 12. In this preferred embodiment, the medicament container 10 has a back sticker 13 mounted on the rear surface of the container main body 11 and having release paper, so the medicament container 10 can be stuck on items or a human body via the back sticker 13 after the release paper is removed. Or, the back sticker 13 can be replaced by a band, so the medicament container 10 can be mounted on items or a human body via the band. The medicament container 10 is capable of assembling with the injection module 2 via the container connecting port 12.

With reference to Figs. 1, 2, 4A, and 4B, the injection module 2 has the conversion mechanism 20 and a needle output mechanism 30. The conversion mechanism 20 and the needle output mechanism 30 can be modular for facilitating assembling the injection module 2 with the medicament container 10 and the driving mechanism 40. A rear surface of the injection module 2 can be implemented with a back sticker having release paper, so that the injection module 2 can be stuck on items or a human body via the back sticker after the release paper is removed, or the back sticker can also be replaced by band, and the injection module 2 is mounted on items or a human body through the band.

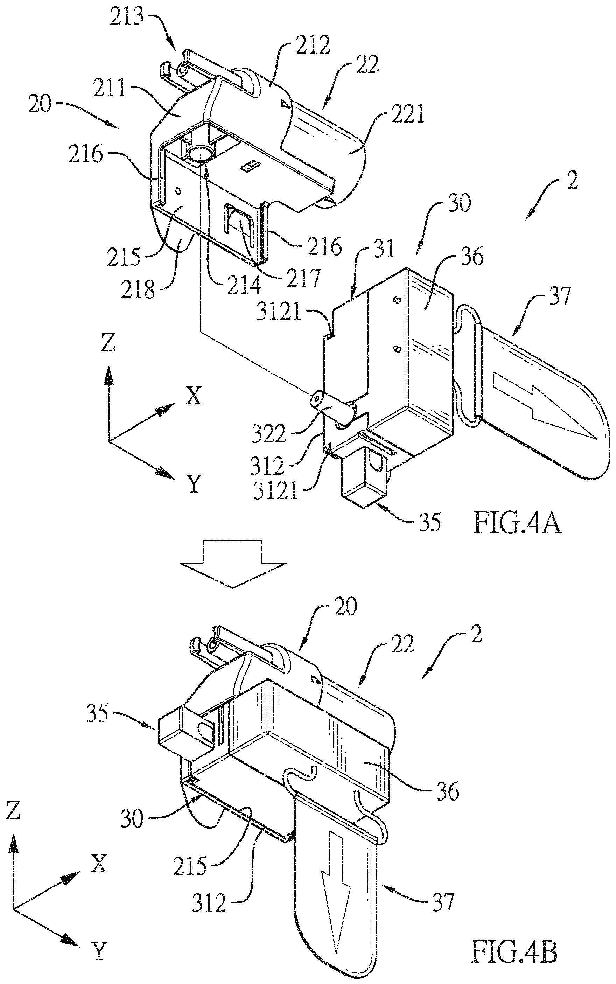

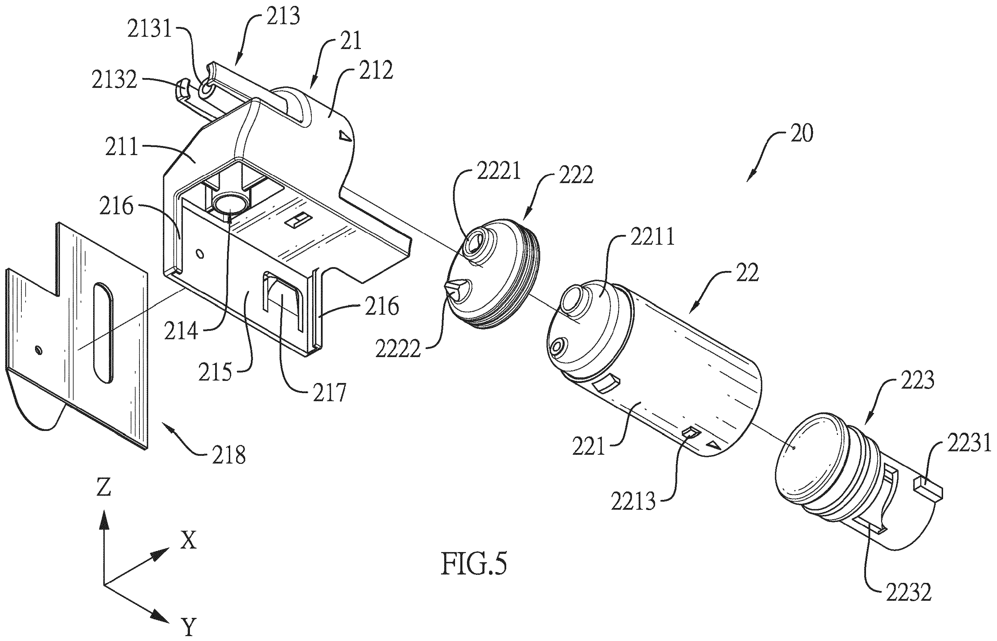

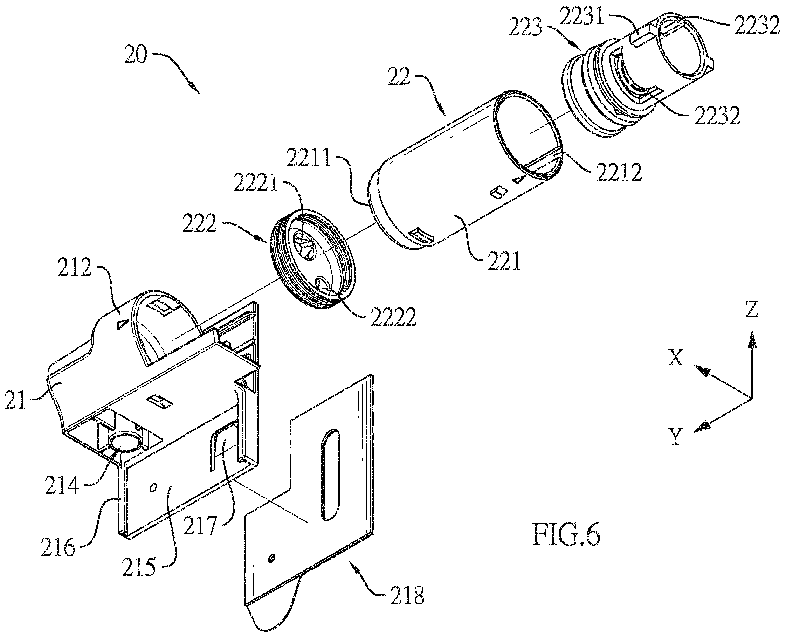

With reference to Figs. 4A, 4B, and 5 to 6, the conversion mechanism 20 is connected to the medicament container 10 and the driving mechanism 40. The conversion mechanism 20 has a conversion connecting base 21 and the driving piston set 22. The conversion connecting base 21 has an input adapter 213 and an output adapter 214, and is connected to the medicament container 10 via the input adapter 213, and is connected to the needle output mechanism 30 via the output adapter 214. In this preferred embodiment, the conversion connecting base 21 has a base body 211 and a valve base segment 212. The valve base segment 212 is mounted on the base body 211. The valve base segment 212 has a sleeving opening toward outside. The sleeving opening is assembled with the driving piston set 22. The input adapter 213 is mounted on a side of the valve base segment 212 opposite to the sleeving opening. The output adapter 214 is mounted in the base body 211. The input adapter 213 and the output adapter 214 are connected to the sleeving opening of the valve base segment 212. The input adapter 213 is detachably connected to the container connecting port 12 of the medicament container 10. The output adapter 214 is connected to the needle output mechanism 30.

With reference to Figs. 4A, 4B, and 5 to 6, in this preferred embodiment, the sleeving opening of the valve base segment 212 faces outwards along the Y axis. The input adapter 213 faces along the Y axis. The output adapter 214 faces along the Z axis. The driving piston set 22 is mounted in the sleeving opening of the valve base segment 212. The driving piston set 22 can be controlled to move and is capable of drawing and transporting the medicament from the medicament container 10 to the driving piston set 22, and then the medicament is output from the output adapter 214. The base body 211 has a connecting board 215 and is connected to the needle output mechanism 30 via the connecting board 215. The connecting board 215 has two clamping segments 216 located respectively on two opposite sides of the connecting board 215 on the Y axis. The connecting board 215 has a hook segment 217, so the connecting board 215 can engage with the needle output mechanism 30.

With reference to Figs. 4A, 4B, and 5 to 6, in this preferred embodiment, the input adapter 213 of the conversion mechanism 20 has a male connecting port 2131 and at least one buckling hook 2132. The container connecting port 12 of the medicament container 10 has a female connecting port 121 and at least one buckling groove 122. The male connecting port 2131 of the input adapter 213 is matched and assembled with the female connecting port 121 of the container connecting port 12. The buckling hook 2132 of the input adapter 213 is matched and assembled with the buckling groove 122 of the container connecting port 12. The male connecting port 2131 and the female connecting port 121 can be implemented as standard male and female luer taper, which prevents leaking after assembled.

With reference to Figs. 4A, 4B, and 5 to 6, in this preferred embodiment, a rear surface of the connecting board 215 of the base body 211 of the conversion connecting base 21 can be implemented with a back sticker 218 having release paper, so that the connecting board 215 can be stuck on items or a human body via the back sticker after the release paper is removed, or the back sticker can also be replaced with a band, and the connecting board 215 is mounted on items or a human body with the band.

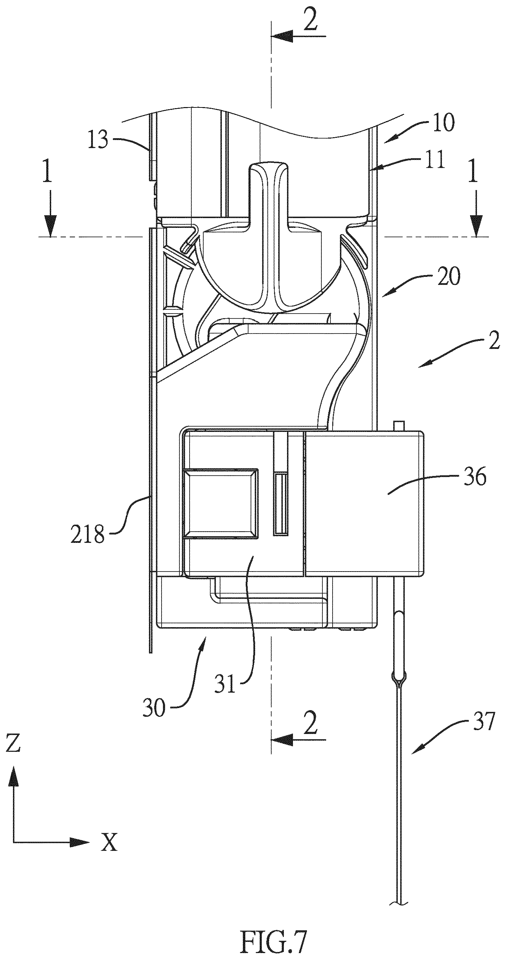

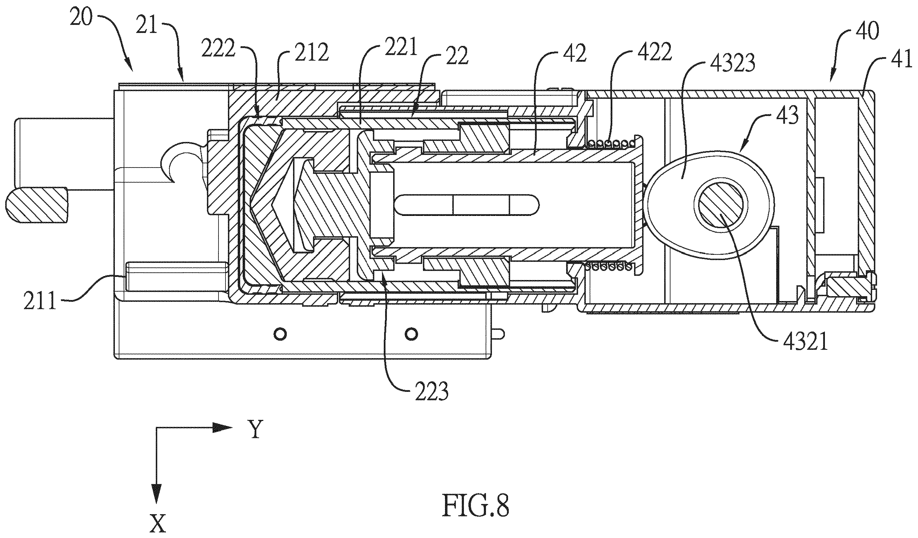

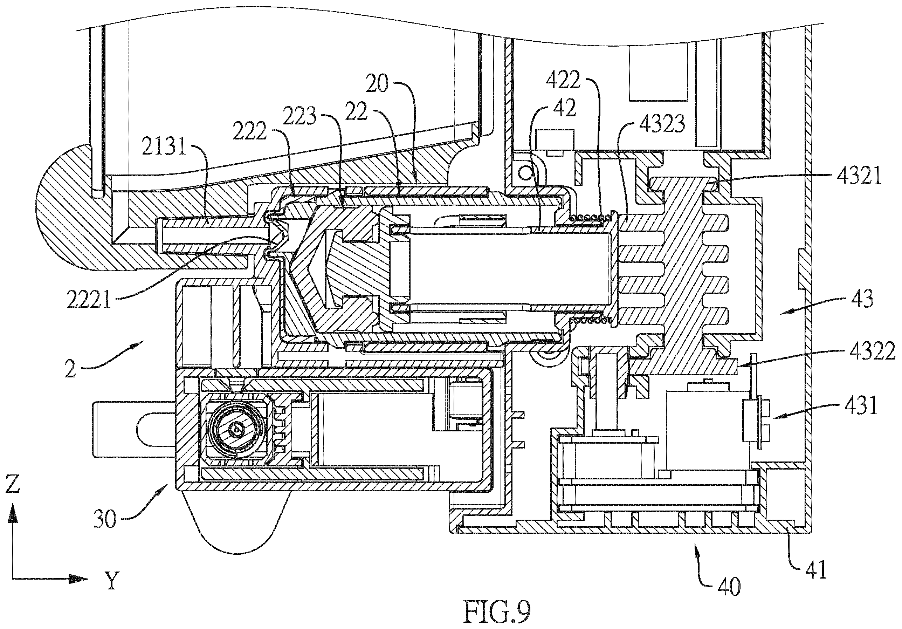

With reference to Figs. 2, 4A, 4B, 5, and 7 to 9, in this preferred embodiment, the driving piston set 22 has a piston cylinder 221, a valve 222, and a piston rod 223 mounted in the piston cylinder 221 and being capable of moving linearly along the Y axis. An end of the piston cylinder 221 has a valve connecting end segment 2211. The piston cylinder 221 has a piston room. The piston room extends along the Y axis to another end of the piston cylinder 221 and forms an opening. The piston cylinder 221 is assembled to the valve 222 via the valve connecting end segment 2211 and is mounted in the valve base segment 212. The valve 222 has an input one-way valve 2221 and an output one-way valve 2222. The input one-way valve 2221 is connected to the input adapter 213. The input one-way valve 2221 is only opened along a direction from the input adapter 213 to the valve base segment 212. The output one-way valve 2222 is connected to the output adapter 214. The output one-way valve 2222 is only opened along a direction from the valve base segment 212 to the output adapter 214. The input adapter 213 and the output adapter 214 of the conversion connecting base 21 are controllably connected to the piston room of the piston cylinder 221 respectively via the input one-way valve 2221 and the output one-way valve 2222 of the valve 222. The piston rod 223 is mounted in the piston cylinder 221 and is capable of moving linearly along the Y axis. The piston rod 223 is detachably connected to the driving mechanism 40, so the piston rod 223 can be driven by the driving mechanism 40.

With reference to Figs. 4A, 4B, and 5 to 6, in this preferred embodiment, an inner cylinder wall of the piston cylinder 221 has at least one sliding groove 2212 extending along the Y axis. An outer annular surface of the piston rod 223 has at least one sliding block 2231. Each of the at least one sliding block 2231 is mounted in a corresponding one of the at least one sliding groove 2212 for guiding the piston rod 223 moving linearly along the Y axis in the piston cylinder 221. Besides, the piston rod 223 is a hollow rod and has at least one L-shaped buckling groove 2232, so the piston rod 223 and the driving mechanism 40 can be assembled by rotating to engage, and thus the piston rod 223 can be driven by the driving mechanism 40.

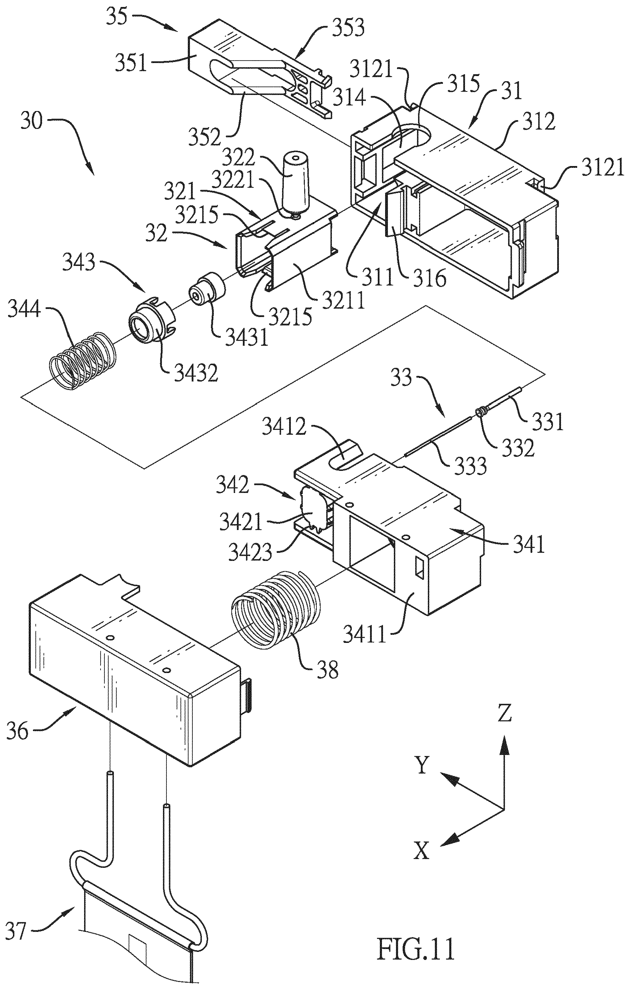

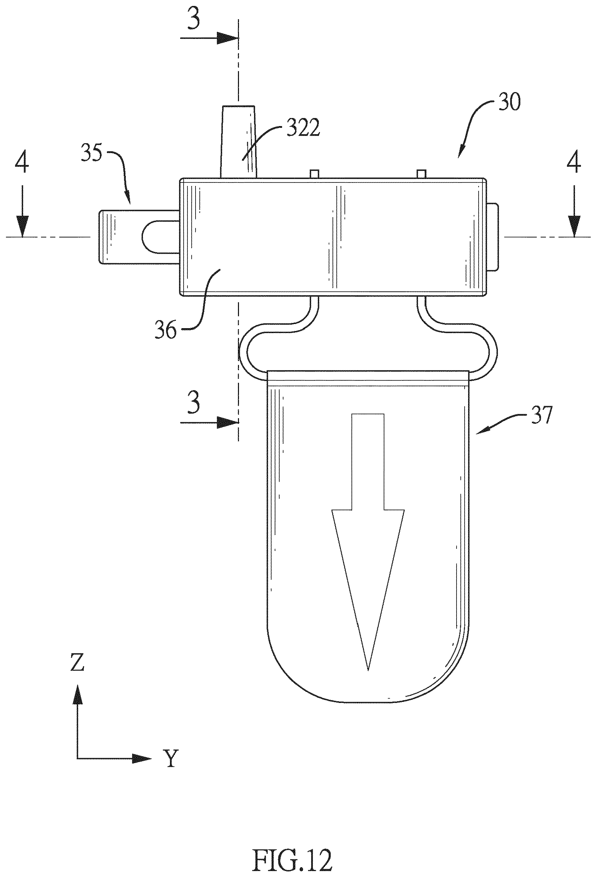

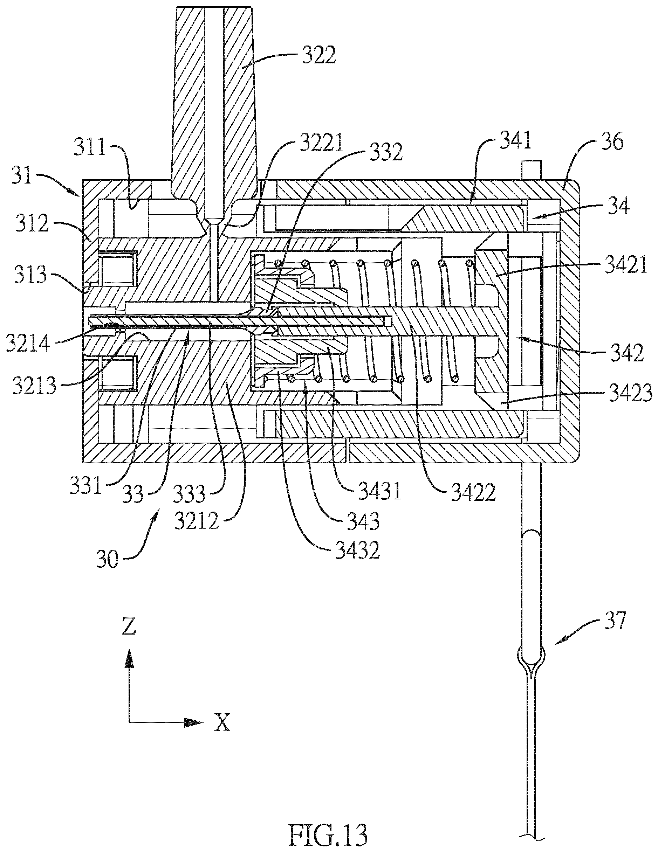

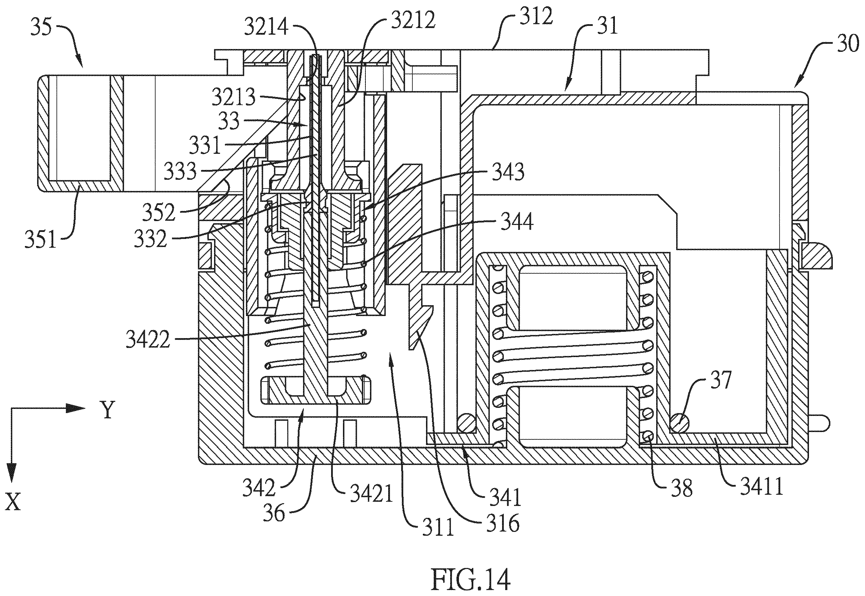

With reference to Figs. 2, 4A, 4B and 10 to 14, the needle output mechanism 30 is connected to the conversion mechanism 20 and is used for medicament injection. The needle output mechanism 30 has a base 31, a connecting unit 32, a needle set 33, a needle output operating assembly 34, and a needle returning operating unit 35.

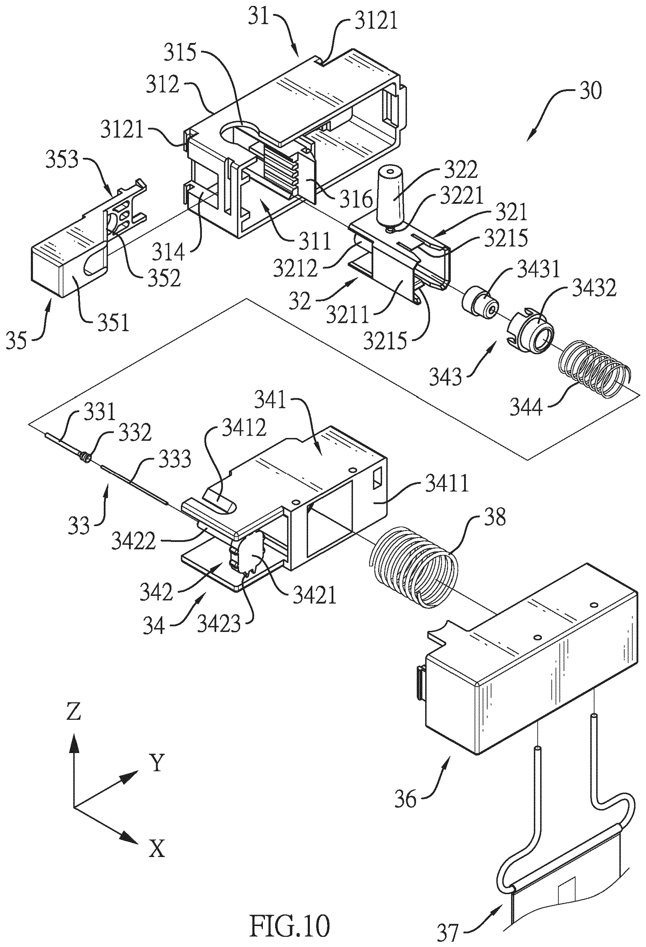

With reference to Figs. 10 to 14, the base 31 can be assembled with the base body 211 of the conversion mechanism 20. The base 31 has an action room 311 with an opening facing a front side along the X axis. The action room 311 has a base board 312 facing a rear side along the X axis. The base board 312 has a penetration hole 313 connected to the action room 311. The base 31 has a through hole 314 formed on a side of the action room 311 along the Y axis and connected to the action room 311. The base 31 has an action opening 315 formed on a side facing the conversion mechanism 20 and connected to the action room 311. The base board 312 has two assembling segments 3121 located on two opposite sides along the Y axis of the base board 312. The clamping segment 216 of the connecting board 215 of the conversion connecting base 21 of the conversion mechanism 20 is assembled with the assembling segment 3121. The hook segment 217 of the connecting board 215 is capable of engaging with the base board 312. The base 31 has a fixing buckling hook 316 mounted on an outer side of the action room 311.

With reference to Figs. 10 to 14, the connecting unit 32 is mounted in the action room 311 of the base 31 and is capable of moving along the X axis. The connecting unit 32 has a connecting base segment 321 and a connecting head 322. The connecting base segment 321 has a connecting base annular wall 3211 and a needle set mounting segment 3212 mounted in the connecting base annular wall 3211. The needle set mounting segment 3212 has a needle room 3213 inside. An end of the needle set mounting segment 3212 facing the base board 312 of the base 31 has a needle plug hole 3214. The needle plug hole 3214 corresponds to the penetration hole 313 of the base board 312. Meanwhile, the needle plug hole 3214 corresponds in position to the penetration hole 313. The connecting head 322 is mounted on the connecting base segment 321 and is connected to the needle room 3213 of the needle set mounting segment 3212. The connecting head 322 extends along the Z axis. In this preferred embodiment, an end of the connecting head 322 connected to the connecting base segment 321 forms a diameter reducing segment 3221 having a reducing diameter and adapted to be cut off. The connecting head 322 of the connecting unit 32 is mounted through the action opening 315 of the base 31 and is connected to the output adapter 214 of the conversion mechanism 20. The connecting base annular wall 3211 has at least one cutting blade segment 3215 located on a side of the connecting base annular wall 3211 away from the base board 312 of the base 31.

With reference to Figs. 10 to 14, the needle set 33 is mounted in the needle set mounting segment of the connecting unit 32, is capable of moving along the X axis, and can be operated to output and retract the needle. The needle set 33 has a soft needle 331, a needle plug 332, and a hard needle 333. The soft needle 331 is mounted in the needle room 3213 of the needle set mounting segment 3212 and is capable of moving along the X axis. An end of the soft needle 331 faces the needle plug hole 3214. Another end of the soft needle 331 is fixed to the needle plug 332. When the soft needle 331 is driven to protrude out of the base board 312 of the base 31, the needle plug 332 is stuck in the needle plug hole 3214. The hard needle 333 can be driven to be mounted in the needle plug 332 and the soft needle, and can protrude along with the soft needle 331. After the soft needle 331 is output, the hard needle 333 can be driven to detach from the soft needle 331, and then the soft needle 331 is connected to the conversion mechanism 20 via the needle room 3213 and the connecting head 322 for providing flow path for the medicament. The soft needle 331 can be retracted to the base 31 along with the connecting unit 32.

With reference to Figs. 10 to 14, the needle output operating assembly 34 is mounted in the base 31 and is capable of driving the needle set 33 to output the needle. The needle output operating assembly 34 has a needle output operating unit 341, a needle base 342, a sealing unit 343, and an elastic unit 344.

With reference to Figs. 10 to 14, the needle output operating unit 341 is mounted in the base 31 and is capable of moving along the X axis. The needle output operating unit 341 can be buckled and fixed by the fixing buckling hook 316 of the base 31. The needle output operating unit 341 has a button segment 3411 and an accommodating space located on a side of the button segment 3411. The accommodating space corresponds to the action room 311 of the base 31. Meanwhile, the accommodating space corresponds in position to the action room 311. A side of the needle output operating unit 341 corresponding to the connecting head 322 has a cutting knife segment 3412. The cutting knife segment 3412 can be operated to cut off the diameter reducing segment 3221 of the connecting head 322 of the connecting unit 32.

With reference to Figs. 10 to 14, the needle base 342 is mounted in the action room 311 of the needle output operating unit 341 and is connected to the needle output operating unit 341. After the needle is output, the needle base 342 is capable of being detached from the needle output operating unit 341. The needle base 342 has a needle base head end 3421 and a needle base connecting rod 3422. The needle base head end 3421 is connected to the needle output operating unit 341. The needle base connecting rod 3422 is formed on the needle base head end 3421. The needle base connecting rod 3422 is connected to the hard needle 333 and is capable of pushing the needle plug 332 of the needle set 33.

With reference to Figs. 10 to 14, in this preferred embodiment, the needle base 342 and the needle output operating unit 341 are formed integrally. A connecting segment 3423, which is adapted to be cut off, is formed between an edge of the needle base head end 3421 and the needle output operating unit 341. By the cutting blade segment 3215 of the connecting unit 32 mounted in the base 31 cutting off the connecting segment 3423, the needle base 342 and the needle output operating unit 341 are detached.

With reference to Figs. 10 to 14, the sealing unit 343 is assembled with the elastic unit 344 and is mounted in the connecting unit 32. The sealing unit 343 is mounted outside the needle set mounting segment 3212 and covers the needle room 3213. The sealing unit 343 sleeves and fits the needle base connecting rod 3422. The elastic unit 344 sleeves the needle base connecting rod 3422. Two ends of the elastic unit 344 are respectively connected to the needle base head end 3421 of the needle base 342 and the sealing unit 343. In this preferred embodiment, the sealing unit 343 has a sealing gasket 3431 and a sleeving cover 3432 sleeving the sealing gasket 3431. The elastic unit 344 abuts the sleeving cover 3432. The sealing gasket 3431 sleeves and fits the needle base connecting rod 3422.

With reference to Figs. 10 to 14, after the needle output operating unit 341 is pressed toward the base 31, the hard needle 333 of the needle set 33 is mounted in the soft needle 331, so the needle output operating unit 341 drives the needle base 342 to push the needle set to protrude out of the base board 312 of the base 31 and press the connecting unit 32 via the elastic unit 344, and thus makes the connecting unit 32 abut the base board 312 located on the rear surface of the base 31, such that the soft needle 331 can pierce into a human body with the hard needle 333 mounted inside. After the needle plug 332 is stuck in the needle plug hole 3214 of the needle set mounting segment 3212, the needle output operating unit 341 is buckled and fixed by the fixing buckling hook 316 in the base 31. The connecting segment 3423 on the edge of the needle base 342 is cut off by the cutting blade segment 3215 of the connecting unit 32. The needle base 342 is pushed out by the elastic unit 344, and then the hard needle 333 is driven to be pulled out of the soft needle 331 by the needle base connecting rod 3422 of the needle base 342, such that the soft needle 331 is connected to the conversion mechanism 20 via the needle room 3213 of the connecting unit and the connecting head 322.

With reference to Figs. 10 to 14, the needle returning operating unit 35 extends from the outer side of the base 31 into the action room 311 via a through hole. The needle returning operating unit 35 has a pressing segment 351, an abutting inclined surface 352 and an extending segment 353. The pressing segment 351 is located outside the base 31. The abutting inclined surface 352 is located on the side where the pressing segment 351 extends into the action room 311. The abutting inclined surface 352 abuts the connecting base annular wall 3211 of the connecting unit 32. The extending segment 353 is located on the side where the pressing segment 351 extends into the action room 311 and bypasses the needle set mounting segment 3212 of the connecting unit 32. A position limiting hook is formed on an end of the extending segment 353. The base 31 limits the position of the position limiting hook such that the needle returning operating unit 35 is mounted in the base 31 and is restricted to move within a limited distance without detaching the base 31. When the pressing segment 351 of the needle returning operating unit 35 is pressed, the abutting inclined surface 352 pushes the connecting unit 32 in the base 31 away from the base board 312 on the back of the base 31, thereby retracting the connecting unit 32 and the soft needle 331 of the needle set 33 mounted in the connecting unit 32 back into the base 31.

With reference to Figs. 10, 11 and 14, the needle output mechanism 30 may be further implemented with a safety cover 36 and a safety bolt 37 to ensure the safety of the needle output mechanism 30. The safety cover 36 covers the needle output operating assembly 34 and is capable of engaging the base 31 for fixing. The safety bolt 37 is mounted in the safety cover 36 and the needle output operating unit 341 of the needle output operating assembly 34. Thus, the needle output mechanism 30 is allowed to output needles only after the safety bolt 37 and the safety cover 36 are removed for ensuring the safety of use. In addition, a pushing elastic unit 38 is mounted between the safety cover 36 and the needle output operating unit 341, so after the safety bolt 37 is pulled out, the safety cover 36 can be automatically pushed away from the base 31 by the pushing elastic unit 38, thereby facilitating operation.

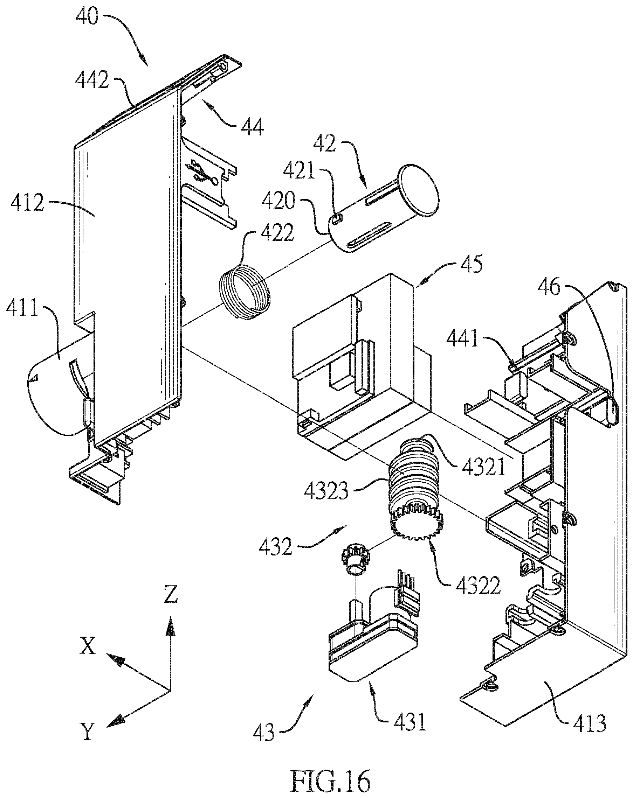

With reference to Figs. 1, 2, 15, and 16, the driving mechanism 40 is assembled with the conversion mechanism 20 of the injection module 2, and is capable of driving the movement of the piston rod 223 in the conversion mechanism 20. The driving mechanism 40 can be a manual driving mechanism or an electric driving mechanism. Preferably, the driving mechanism 40 is detachably connected to the conversion mechanism 20 of the injection module 2, so that the driving mechanism 40 can be reused. The driving mechanism 40 has a case 41 and a driving unit 42 mounted in the case 41. The case 41 is detachably connected to the conversion connecting base 21 of the conversion mechanism 20. The driving unit 42 is detachably connected to the piston rod 223. The driving unit 42 is capable of manually or electrically driving the piston rod 223 to move.

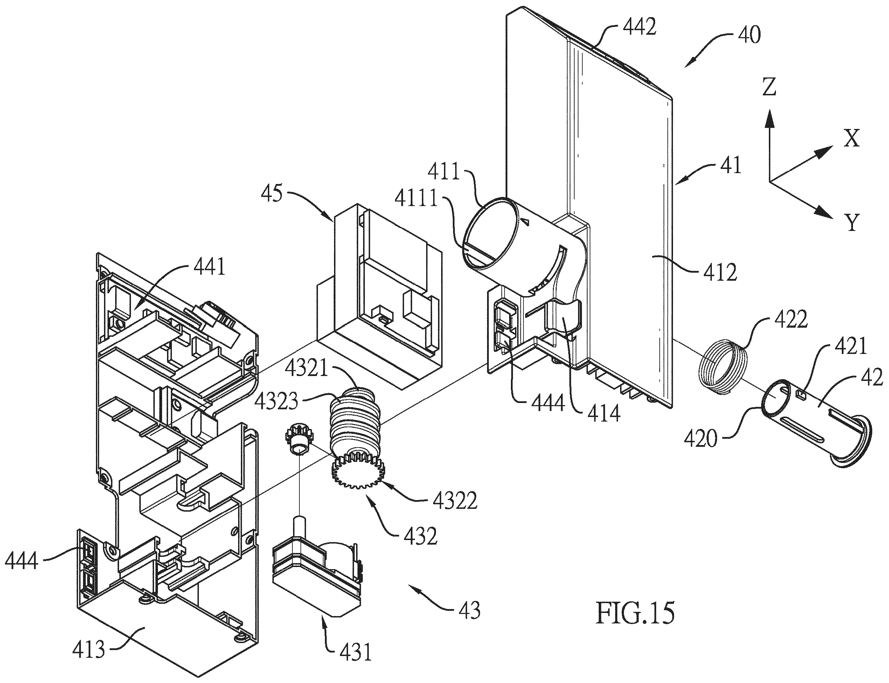

With reference to the preferred embodiment shown in Figs. 15 to 16, the case 41 is a hollow body with a space inside. A side of the case 41 has a connecting sleeve segment 411. The connecting sleeve segment 411 has a shaft hole extending from an outer side into the case 41. The connecting sleeve segment 411 is detachably sleeved on the piston cylinder 221 of the conversion mechanism 20.

With reference to Figs. 15 to 16, in this preferred embodiment, the case 41 is composed of multiple case body segments 412, 413 in a screw locking, clipping or bonding manner. The connecting sleeve segment 411 is rotatably assembled with the piston cylinder 221. An outer peripheral surface of the piston cylinder 221 is implemented with a buckling block 2213, and the connecting sleeve segment 411 is implemented with an L-shaped buckling groove 4111. By the buckling block 2213 and the L-shaped buckling groove 4111, the connecting sleeve segment 411 and the piston cylinder 221 are rotatably assembled with each other.

With reference to Figs. 15 to 16, 8 and 9, the driving unit 42 is mounted in the connecting sleeve segment 411 and is capable of moving linearly. An end of the driving unit 42 facing the connecting sleeve segment 411 forms a connecting end segment 420. The connecting end segment 420 is detachably connected to the piston rod 223. In this preferred embodiment, an outer peripheral surface of the connecting end segment 420 of the driving unit 42 has at least one engaging protrusion 421. The driving unit 42 can be mounted in the piston rod 223 and matches the L-shaped buckling of the engaging protrusion 421 via the engaging protrusion 421, so that the piston rod 223 and the driving mechanism 40 are rotatably assembled with each other.

With reference to Figs. 15 to 16, in this preferred embodiment, the driving mechanism 40 is an electric driving mechanism, but not limited thereto. When the driving mechanism 40 is an electric driving mechanism, the driving mechanism 40 has the case 41 and the driving unit 42 mounted in the case 41, and further has an electrical driving unit 43 and a controlling unit 44, or a power storage unit 45.

With reference to Figs. 15 to 16, the electrical driving unit 43 is mounted in the case 41 and can be connected to the driving unit 42 in the connecting sleeve segment 411 of the case 41. The electrical driving unit 43 has a driving assembly 431 and a transmission assembly 432. The driving assembly 431 can be an electric driving mechanism including a motor and a pump or other electric rotating driving mechanism. The transmission assembly 432 connects the driving assembly 431 and the driving unit 42, so that the driving assembly 431 can drive the driving unit 42 via the transmission assembly 432. In this preferred embodiment, the transmission assembly 432 has a transmission rod 4321 and a gear assembly 4322. The transmission rod 4321 is pivotally mounted in the case 41. The transmission rod 4321 has at least one eccentric cam 4323. The gear assembly 4322 has multiple gears engaging with each other. The gear assembly 4322 is connected between the driving assembly 431 and the transmission rod 4321. The eccentric cam 4323 of the transmission rod 4321 is connected to another end of the driving unit 42 located in the case 41. A retreating elastic unit 422 is mounted between the driving unit 42 and the case 41 for driving the driving unit 42 and the connected piston rod 223 to reciprocate linearly. In this preferred embodiment, the restoring elastic unit 422 is a compressed elastic unit. The restoring elastic unit 422 is sleeved on the driving unit 42. The end of the driving unit 42 located in the case 41 has an abutting flange. Two ends of the retreating elastic unit 422 respectively abut the case 41 and the abutting flange of the driving unit 42.

With reference to Figs. 15 to 16, the controlling unit 44 is mounted in the case 41, and the controlling unit 44 has a controlling circuit board 441 and a display panel 442. The display panel 442 is electrically connected to the controlling circuit board 441. The display panel 442 is exposed from a front surface of the case 41. The display panel 442 can be a conventional display panel having a display function. The controlling unit 44 also has at least one controlling button 443. The controlling button 443 is located on the front surface of the case 41 and is located on a side of the display panel 442. The controlling button 443 is electrically connected to the controlling circuit board 441, so that the user can switch, set and operate via the controlling button 443. Alternatively, the display panel 442 can also be a touch-sensitive display panel. The touch-sensitive display panel not only displays the working status, but also has user operation definition, allowing users to set up and operate on the display panel.

With reference to Figs. 15 to 16, in addition, the controlling unit 44 also has at least one contact switch 444. The contact switch 444 is mounted on a side of the case 41 adjacent to the needle output mechanism 30, and is electrically connected the controlling circuit board 441. When the driving mechanism 40 is connected to the conversion mechanism 20 of the injection module 2, the needle output mechanism 30 of the injection module 2 can touch the contact switch 444 so that the controlling unit 44 is switched from off to on state. The driving mechanism 40 can also be implemented with a fixing hook 414 on the case 41 at a position adjacent to the contact switch 444. The base 31 of the needle output mechanism 30 can be fixed on the case 41 by the fixing hook 414, so that the driving mechanism 40 and the injection module 2 are firmly assembled.

With reference to Figs. 15 to 16, the controlling unit 44 can be connected to an external power supply or the power storage unit 45, and the external power supply or the power storage unit 45 can provide the electric energy required for the driving mechanism 40 to operate. In the preferred embodiment shown in Fig. 15 to Fig. 16, the driving mechanism 40 also has a power storage unit 45 mounted in the case 41 and electrically connected to the controlling circuit of the controlling circuit board 441. The power storage unit 45 can be a wireless rechargeable power storage unit, a wired rechargeable power storage unit or any other non-rechargeable power storage unit (for example: battery). In the preferred embodiment shown in Figs. 15 to 16, the power storage unit 45 is a rechargeable power storage unit, such as a lithium battery. The power storage unit 45 is electrically connected to an electrically connecting unit 46. The electrically connecting unit 46 is exposed from the case 41, and can be connected to an external power source through a connection line for recharging. The electrically connecting unit 46 can be a universal serial bus (Universal Serial Bus, USB) type electrically connecting unit or other types of electrically connecting unit.

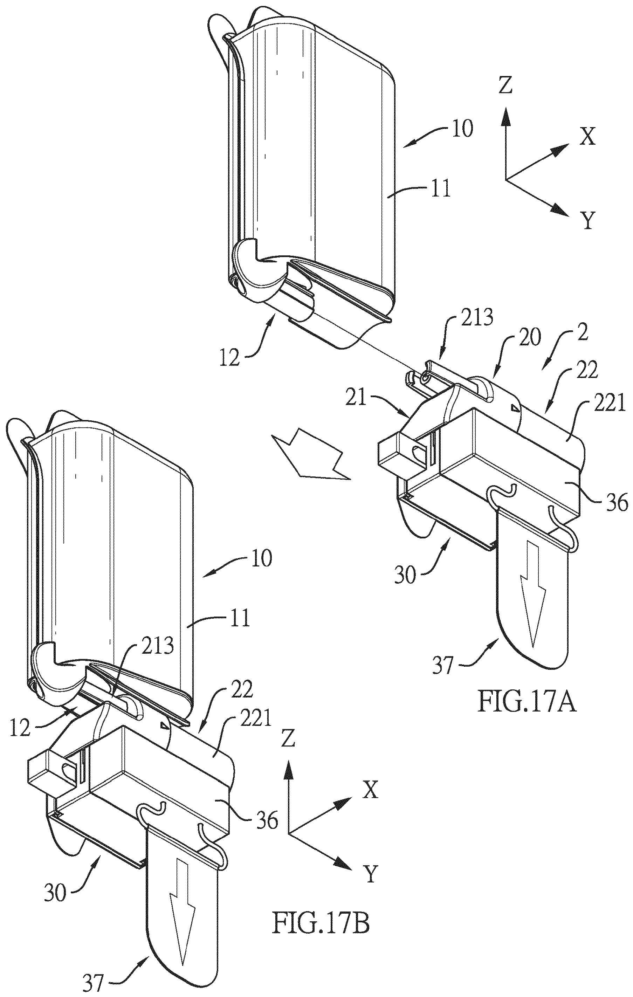

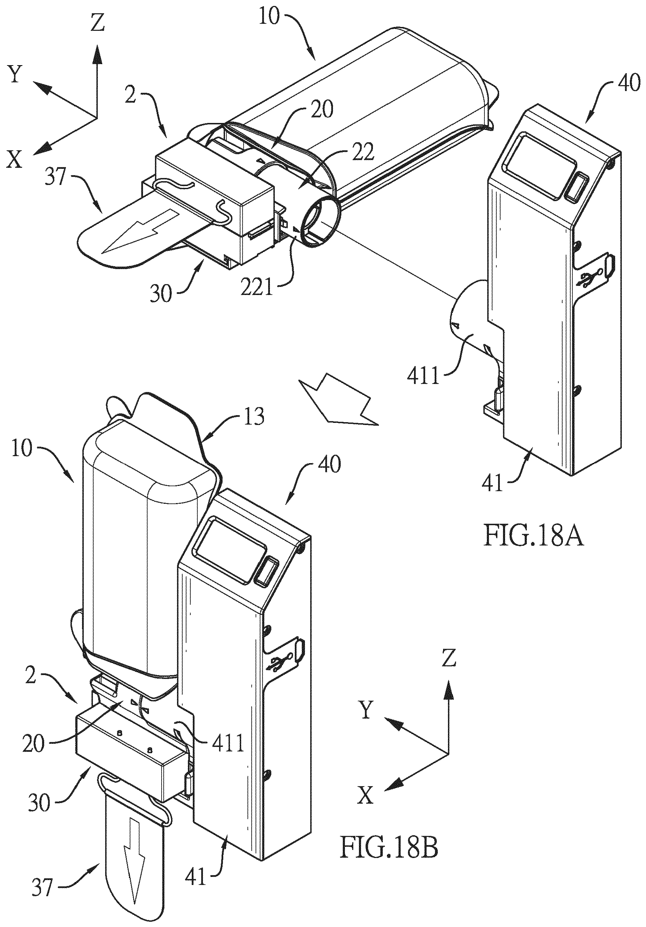

Regarding the use of the injector of the present invention, with reference to Figs. 17A, 17B, 18A and 18B. The conversion mechanism 20 and the needle output mechanism 30 have been pre-assembled into an injection module 2 upon shipping out. Before the user performs subcutaneous injection, the medicament container 10 with the injection medicament and the driving mechanism 40 are sequentially assembled to the conversion mechanism 20 of the injection module 2. Herein, the medicament container 10 is assembled with the input adapter 213 of the conversion mechanism 20 of the injection module 2 via the container connecting port 12, and then the driving mechanism 40 is connected to the conversion mechanism 20. Wherein, the connecting sleeve segment 411 of the driving mechanism 40 is connected to the piston cylinder 221 of the conversion mechanism 20 by rotational engagement. At the same time, the driving unit is connected to the piston rod 223. After the injection module 2 rotates to a predetermined position, the needle output mechanism 30 contacts the contact switch 444 of the driving mechanism 40, such that the controlling unit 44 of the driving mechanism 40 is switched from the off state to the on state.

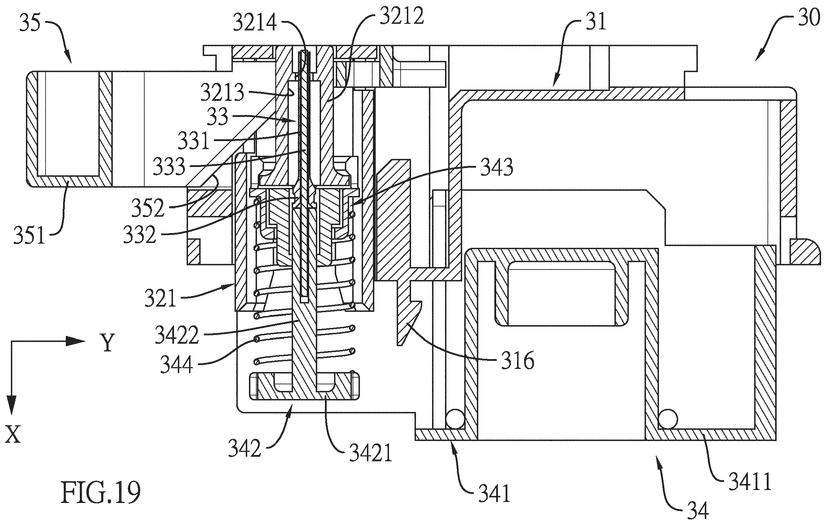

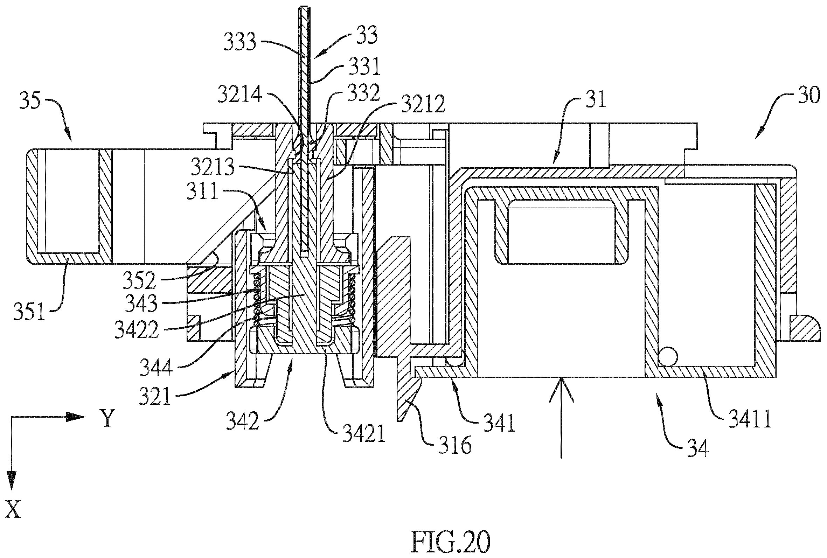

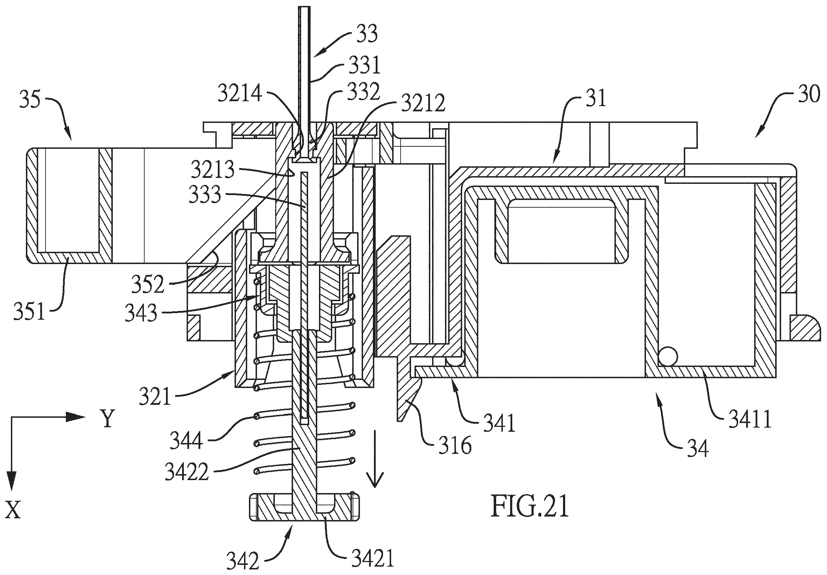

With reference to Figs. 18A and 18B to 21, before injection, the medicament container 10 and the injection module 2 are fixed on the intended injection site of the human body by the back sticker 13 or strap, and then remove the safety bolt 37 and safety cover 36 of the needle output mechanism 30 of the injection module 2. Next, press the needle output operating unit 341 of the needle output mechanism 30 to fix the needle output operating unit 341 on the base 31 and to push the needle set 33 via the needle base 342 and make the needle set 33 protrude with the hard needle 333 mounted in the soft needle 331 and pierce directly into the human body. On the other hand, the connecting segment 3423 between the needle base 342 and the needle output operating unit 341 is cut off by the cutting blade segment 3215 of the connecting unit 32. The elastic unit 344 pushes the needle base 342 and pulls out the hard needle 333 from the soft needle 331. The soft needle 331 is left in the human body and is connected to the output adapter 214 of the conversion mechanism 20 via the needle room 3213 and the connecting head 322. Later, the user is allowed to operate the driving mechanism 40 to drive the driving piston set 22 of the conversion mechanism 20 for drawing the medicament from the medicament container 10, conveyed to the needle output mechanism 30, and injected into the human body via the soft needle 331. After the injection is completed, stop the driving mechanism 40 from driving the driving piston set 22 of the conversion mechanism 20. In this way, with the injector fixed on the human body and the soft needle 331 staying in the state of piercing the human body, the user can regularly perform multiple injections through the driving mechanism 40.

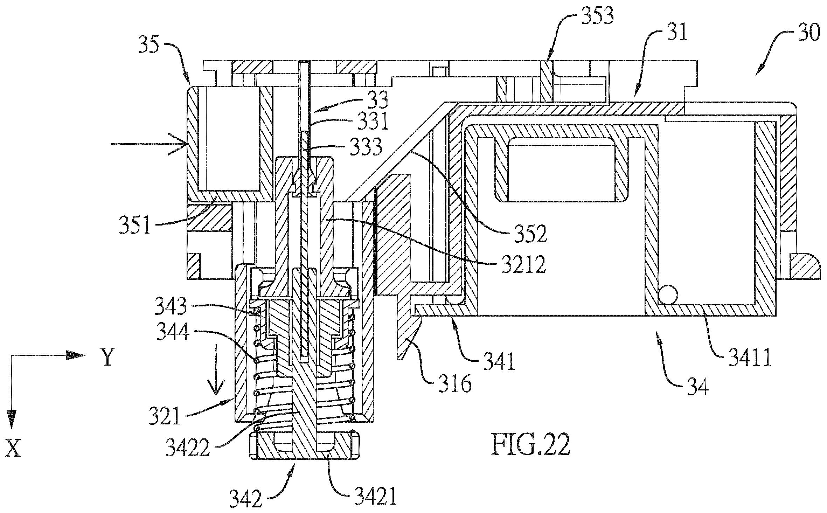

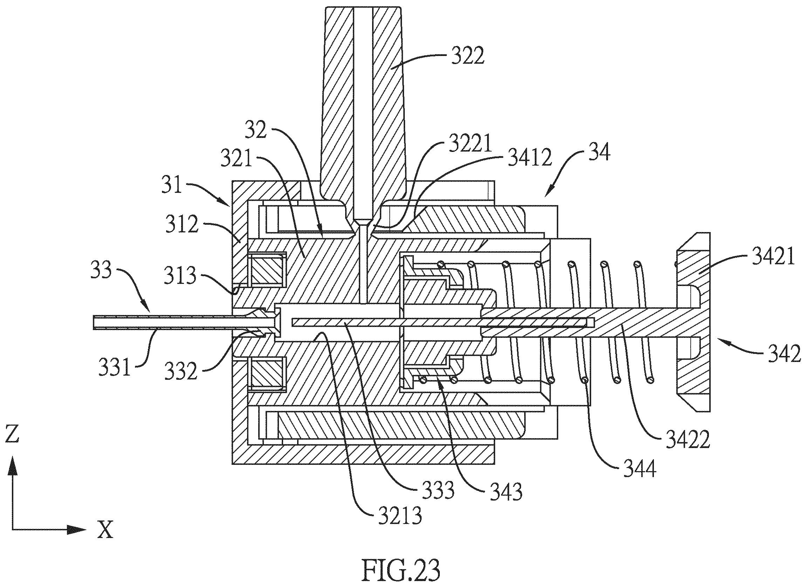

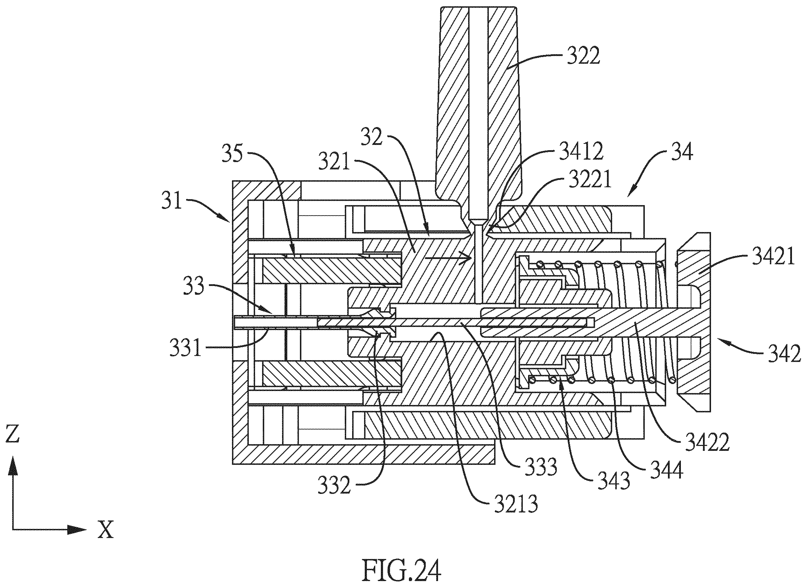

After the medicament in the medicament container 10 is used up, as shown in Fig. 22, the user can press the needle return operating unit 35 of the needle output mechanism 30 and use the abutting inclined surface 352 of the needle return operating unit 35 to push the connecting unit 32 in the base 31 away from the base board 312 on the back of the base 31, and at the same time drives the soft needle 331 of the needle set 33 in the connecting unit 32 to be pulled out from the human body and retracted into the base 31 together. With reference to Figs. 23 and 24, at this time, while the abutting inclined surface 352 of the needle returning operating unit 35 is pushing the connecting unit 32, the diameter reducing segment 3221 of the connecting head 322 connected to the connecting unit 32 is cut off by the cutting knife segment 3412 of the needle output operating unit 341 fixed on the base 31, such that the injection module 2 cannot be reused to ensure the safety of the injector. After the injector is used, the driving mechanism 40 can be removed from the injection module 2 for another use.

## Claims

1. A driving piston set (22) of an injector, configured to be used in the injector and be connected to a driving mechanism (40), and the driving piston set (22) having:
a piston cylinder (221) having
a piston room; and
a valve connecting end segment (2211) defined on an end of the piston cylinder (221);
a valve (222) assembled to the valve connecting end segment (2211) of the piston cylinder (221) and having
an input one-way valve (2221) being only opened in a direction towards the piston cylinder (221); and
an output one-way valve (2222) being only opened in a direction outward from the piston cylinder (221); and
a piston rod (223) mounted in the piston cylinder (221) and being linearly movable, and detachably connected to the driving mechanism (40);
the driving piston set (22) **characterized in that**:
an inner cylinder wall of the piston cylinder (221) has at least one sliding groove (2212); and
an outer annular surface of the piston rod (223) has at least one sliding block (2231), each of the at least one sliding block (2231) is mounted in a corresponding one of the at least one sliding groove (2212) for guiding the piston rod (223) to move linearly in the piston cylinder (221)

2. The driving piston set (22) of the injector as claimed in claim 1, wherein the piston rod (223) is a hollow rod and has at least one L-shaped buckling groove (2232), so the piston rod (223) and the driving mechanism (40) is assembled by rotating to engage and the piston rod (223) is driven by the driving mechanism (40).

3. A conversion mechanism (20) of an injector, configured to be used in the injector and be connected to a driving mechanism (40) having a driving unit (42), and the conversion mechanism (20) **characterized in** having:
a conversion connecting base (21) having an input adapter (213) and an output adapter (214); and
a driving piston set (22) according to any of the previous claims.

4. The conversion mechanism (20) of the injector as claimed in claim 3, wherein
the conversion mechanism (20) is assembled with a needle output mechanism (30) having a needle set (33) to form an injection module (2);
the input adapter (213) of the conversion mechanism (20) is connected to a medicament container (10) containing medicament;
the output adapter (214) of the conversion mechanism (20) is connected to the needle output mechanism (30); and
the driving mechanism (40) drives the piston rod (223) in the piston cylinder (221) to draw the medicament from the medicament container (10) to the needle output mechanism (30) and to inject the medicament via the needle set (33) of the needle output mechanism (30).

5. The conversion mechanism (20) of the injector as claimed in claim 4, wherein
an inner cylinder wall of the piston cylinder (221) has at least one sliding groove (2212); and
an outer annular surface of the piston rod (223) has at least one sliding block (2231), each of the at least one sliding block (2231) is mounted in a corresponding one of the at least one sliding groove (2212) for guiding the piston rod (223) to move linearly in the piston cylinder (221).

6. The conversion mechanism (20) of the injector as claimed in claim 4, wherein the piston rod (223) is a hollow rod and has at least one L-shaped buckling groove (2232), so the piston rod (223) and the driving mechanism (40) is assembled by rotating to engage and the piston rod (223) is driven by the driving mechanism (40).

7. The conversion mechanism (20) of the injector as claimed in any one of claims 4 to 6, wherein
the conversion connecting base (21) has
a base body (211); and
a valve base segment (212) mounted on the base body (211) and having a sleeving opening;
the input adapter (213) is mounted on a side of the valve base segment (212) opposite to the sleeving opening; and
the output adapter (214) is mounted in the base body (211);
wherein the input adapter (213) and the output adapter (214) are connected to the sleeving opening.

8. The conversion mechanism (20) of the injector as claimed in claim 7, wherein the base body (211) has a connecting board (215), and the connecting board (215) has two clamping segments (216) located respectively on two opposite sides of the connecting board (215) and a hook segment (217), so the connecting board (215) engages with the needle output mechanism (30).

9. The conversion mechanism (20) of the injector as claimed in claim 7, wherein the driving mechanism (40) has a case (41), the driving unit (42) is mounted in the case (41), a side of the case (41) has a connecting sleeve segment (411) detachably sleeved on the piston cylinder (221), and the driving unit (42) is linearly movable in the connecting sleeve segment (411).

## Patentansprüche

1. - Antriebskolbensatz (22) eines Injectors, die zur Verwendung in dem Injector und zum Anschluss an einen Antriebsmechanismus (40) ausgelegt ist, wobei der Antriebskolbensatz (22) aufweist:
einen Kolbenzylinder (221) mit
einem Kolbenraum; und
einem an einem Ende des Kolbenzylinders (221) ausgebildetes Ventilverbindungsendsegment (2211);
ein Ventil (222), das an dem Ventilverbindungsendsegment (2211) des Kolbenzylinders (221) angebracht ist und Folgendes aufweist:
ein Einweg-Eingangventil (2221), das nur in Richtung des Kolbenzylinders (221) geöffnet ist; und
ein Einweg-Ausgangventil (2222), das nur in Richtung aus dem Kolbenzylinder (221) heraus geöffnet ist; und
eine Kolbenstange (223), die im Kolbenzylinder (221) montiert und linear beweglich ist und lösbar mit dem Antriebsmechanismus (40) verbunden ist;
wobei der Antriebskolbensatz (22) **dadurch gekennzeichnet ist, dass**:
eine innere Zylinderwand des Kolbenzylinders (221) mindestens eine Gleitnut (2212) aufweist; und
eine äußere Ringfläche der Kolbenstange (223) mindestens einen Gleitblock (2231) aufweist, wobei jeder des mindestens einen Gleitblocks (2231) in einer entsprechenden des mindestens einen Gleitnut (2212) montiert ist, um die Kolbenstange (223) so zu führen, dass sie sich linear im Kolbenzylinder (221) bewegt.

2. - Antriebskolbensatz (22) des Injektors nach Anspruch 1, wobei die Kolbenstange (223) eine Hohlstange ist und mindestens eine L-förmige Rastnut (2232) aufweist, so dass die Kolbenstange (223) und der Antriebsmechanismus (40) durch Drehen in Eingriff gebracht werden und die Kolbenstange (223) durch den Antriebsmechanismus (40) angetrieben wird.

3. - Umwandlungsmechanismus (20) eines Injektors, der zur Verwendung in dem Injektor und zum Anschluss an einen Antriebsmechanismus (40) mit einer Antriebseinheit (42) ausgelegt ist, wobei der Umwandlungsmechanismus (20) **dadurch gekennzeichnet ist, dass** er aufweist:
eine Umwandlungsverbindungsbasis (21) mit einem Eingangsadapter (213) und einem Ausgangsadapter (214); und
einen Antriebskolbensatz (22) gemäß einem der vorstehenden Ansprüchen.

4. - Umwandlungsmechanismus (20) des Injektors nach Anspruch 3, wobei
der Umwandlungsmechanismus (20) ist mit einem Nadelausgangmechanismus (30) verbunden, der einen Nadelsatz (33) aufweist, um ein Injektionsmodul (2) zu bilden;
der Eingangsadapter (213) des Umwandlungsmechanismus (20) ist mit einem Medikamentbehälter (10) verbunden, der ein Medikament enthält;
der Auslassadapter (214) des Umwandlungsmechanismus (20) ist mit dem Nadelausgangmechanismus (30) verbunden; und
der Antriebsmechanismus (40) treibt die Kolbenstange (223) im Kolbenzylinder (221) an, um das Medikament aus dem Medikamentbehälter (10) zum Nadelausgangmechanismus (30) anzusaugen und das Medikament über den Nadelsatz (33) des Nadelausgangmechanismus (30) zu injizieren.

5. - Umwandlungsmechanismus (20) des Injektors nach Anspruch 4, wobei
eine innere Zylinderwand des Kolbenzylinders (221) mindestens eine Gleitnut (2212) aufweist; und
eine äußere Ringfläche der Kolbenstange (223) mindestens einen Gleitblock (2231) aufweist, wobei jeder des mindestens einen Gleitblocks (2231) in einer entsprechenden der mindestens einen Gleitnut (2212) montiert ist, um die Kolbenstange (223) so zu führen, dass sie sich linear im Kolbenzylinder (221) bewegt.

6. - Umwandlungsmechanismus (20) des Injektors nach Anspruch 4, wobei die Kolbenstange (223) eine Hohlstange ist und mindestens eine L-förmige Rastnut (2232) aufweist, so dass die Kolbenstange (223) und der Antriebsmechanismus (40) durch Drehen in Eingriff gebracht werden und die Kolbenstange (223) durch den Antriebsmechanismus (40) angetrieben wird.

7. - Umwandlungsmechanismus (20) des Injektors nach einem der Ansprüche 4 bis 6, wobei
die Umwandlungsverbindungsbasis (21) aufweist:
einen Basiskörper (211) ; und
ein Ventilbasissegment (212), das an dem Basiskörper (211) angebracht ist und eine Hülsenöffnung aufweist;
der Eingangsadapter (213) an einer der Hülsenöffnung gegenüberliegenden Seite des Ventilbasissegments (212) angebracht ist; und
der Ausgangsadapter (214) im Basiskörper (211) angebracht ist;
wobei der Eingangsadapter (213) und der Ausgangsadapter (214) mit der Hülsenöffnung verbunden sind.

8. - Umwandlungsmechanismus (20) des Injektors nach Anspruch 7, wobei der Basiskörper (211) eine Verbindungsplatte (215) aufweist und die Verbindungsplatte (215) zwei Klemmsegmente (216), die sich jeweils an zwei gegenüberliegenden Seiten der Verbindungsplatte (215) befinden, sowie ein Hakensegment (217) aufweist, so dass die Verbindungsplatte (215) mit dem Nadelausgangmechanismus (30) in Eingriff steht.

9. - Umwandlungsmechanismus (20) des Injektors nach Anspruch 7, wobei der Antriebsmechanismus (40) ein Gehäuse (41) aufweist, die Antriebseinheit (42) in dem Gehäuse (41) angebracht ist, eine Seite des Gehäuses (41) ein Verbindungshülsenelement (411) aufweist, das lösbar auf den Kolbenzylinder (221) aufgeschoben ist, und die Antriebseinheit (42) in dem Verbindungshülsenelement (411) linear verschiebbar ist.

## Revendications

1. - Ensemble piston d'entraînement (22) d'un injecteur, configuré pour être utilisé dans l'injecteur et être relié à un mécanisme d'entraînement (40), et l'ensemble piston d'entraînement (22 ayant :
un cylindre de piston (221) ayant
une chambre de piston ; et
un segment d'extrémité de liaison de valve (2211) défini sur une extrémité du cylindre de piston (221) ;
une valve (222) assemblée au segment d'extrémité de liaison de valve (2211) du cylindre de piston (221) et ayant
un clapet de non-retour d'entrée (222) qui est uniquement ouvert dans une direction allant vers le cylindre de piston (221) ; et
un clapet de non-retour de sortie (222) qui est uniquement ouvert dans une direction allant à l'opposé du cylindre de piston (221) ; et
une tige de piston (223) montée dans le cylindre de piston (221) et mobile linéairement, et reliée de manière détachable au mécanisme d'entraînement (40) ;
l'ensemble piston d'entraînement (22) étant **caractérisé par le fait que** :
une paroi de cylindre intérieure du cylindre de piston (221) a au moins une rainure de coulissement (2212) ; et
une surface annulaire extérieure de la tige de piston (223) a au moins un bloc de coulissement (2231), chacun du au moins un bloc de coulissement (2231) est monté dans l'une, correspondante, de l'au moins une rainure de coulissement (2212) pour guider la tige de piston (223) pour qu'elle se déplace linéairement dans le cylindre de piston (221).

2. - Ensemble piston d'entraînement (22) de l'injecteur selon la revendication 1, dans lequel la tige de piston (223) est une tige creuse et a au moins une rainure d'emboîtage élastique en forme de L (2232), de telle sorte que la tige de piston (223) et le mécanisme d'entraînement (30) sont assemblés par rotation pour s'engager et la tige de piston (223) est entraînée par le mécanisme d'entraînement (40).

3. - Mécanisme de conversion (20) d'un injecteur, configuré pour être utilisé dans l'injecteur et être relié à un mécanisme d'entraînement (40) ayant une unité d'entraînement (42), et le mécanisme de conversion (20) étant **caractérisé par le fait qu'**il a :
une base de liaison de conversion (21) ayant un adaptateur d'entrée (213) et un adaptateur de sortie (214) ; et
un ensemble piston d'entraînement (22) selon l'une quelconque des revendications précédentes.

4. - Mécanisme de conversion (20) de l'injecteur selon la revendication 3, dans lequel
le mécanisme de conversion (20) est assemblé à un mécanisme de sortie d'aiguille (30) ayant un ensemble aiguille (33) pour former un module d'injection (2) ;
l'adapateur d'entrée (213) du mécanisme de conversion (20) est relié à un contenant à médicament (10) contenant un médicament ;
l'adapateur de sortie (214) du mécanisme de conversion (20) est relié au mécanisme de sortie d'aiguille (30) ; et
le mécanisme d'entraînement (40) entraîne la tige de piston (223) dans le cylindre de piston (221) pour aspirer et transporter du médicament à partir du contenant à médicament (10) jusqu'au mécanisme de sortie d'aiguille (30), et injecter le médicament par l'intermédiaire de l'ensemble aiguille (33) du mécanisme de sortie d'aiguille (30).

5. - Mécanisme de conversion (20) de l'injecteur selon la revendication 4, dans lequel
une paroi de cylindre intérieure du cylindre de piston (221) a au moins une rainure de coulissement (2212) ;
une surface annulaire extérieure de la tige de piston (223) a au moins un bloc de coulissement (2231), chacun du au moins un bloc de coulissement (2231) est monté dans l'une, correspondante, de l'au moins une rainure de coulissement (2212) pour guider la tige de piston (223) pour qu'elle se déplace linéairement dans le cylindre de piston (221).

6. - Mécanisme de conversion (20) de l'injecteur selon la revendication 4, dans lequel la tige de piston (223) est une tige creuse et a au moins une rainure d'emboîtage élastique en forme de L (2232), de telle sorte que la tige de piston (223) et le mécanisme d'entraînement (30) sont assemblés par rotation pour s'engager et la tige de piston (223) est entraînée par le mécanisme d'entraînement (40).

7. - Mécanisme de conversion (20) de l'injecteur selon l'une quelconque des revendications 4 à 6, dans lequel
la base de liaison de conversion (21) a
un corps de base (211) ; et
un segment de base de valve (212) monté sur le corps de base (211) et ayant une ouverture de manchon ;
l'adaptateur d'entrée (213) est monté sur un côté du segment de base de valve (212) qui est opposé à l'ouverture de manchon ; et
l'adaptateur de sortie (214) est monté dans le corps de base (211) ;
l'adaptateur d'entrée (213) et l'adaptateur de sortie (214) étant reliés à l'ouverture de manchon.

8. - Mécanisme de conversion (20) de l'injecteur selon la revendication 7, dans lequel le corps de base (211) a une plaque de liaison (215), et la plaque de liaison (215) a deux segments de serrage (216) situés respectivement sur deux côtés opposés de la plaque de liaison (215) et un segment crochet (217), de telle sorte que la plaque de liaison (215) s'engage avec le mécanisme de sortie d'aiguille (30).

9. - Mécanisme de conversion (20) de l'injecteur selon la revendication 7, dans lequel le mécanisme d'entraînement 40) a un boîtier (41), l'unité d'entraînement (42) est montée dans le boîtier (41), un côté du boîtier (41) a un segment de manchon de liaison (411) emmanché de manière détachable sur le cylindre de piston (221), et l'unité d'entraînement (42) est mobile linéairement dans le segment de manchon de liaison (411).
